(19) Europäisches Patentamt
European Patent Office
Office européen des brevets

(11) EP 3 272 227 B1

(12) **EUROPEAN PATENT SPECIFICATION**

(45) Date of publication and mention of the grant of the patent:
**28.10.2020 Bulletin 2020/44**

(21) Application number: **16764981.3**

(22) Date of filing: **15.03.2016**

(51) Int Cl.:
***A23L 2/02*** *(2006.01)* ***C11B 9/02*** *(2006.01)*

(86) International application number:
**PCT/JP2016/058184**

(87) International publication number:
**WO 2016/148151 (22.09.2016 Gazette 2016/38)**

(54) **LIQUID COMPOSITION INCLUDING CITRUS PERICARP ESSENTIAL OIL**

FLÜSSIGE ZUSAMMENSETZUNG MIT ÄTHERISCHEM ZITRUSÖL AUS DEM PERIKARP

COMPOSITION LIQUIDE COMPRENANT UNE HUILE ESSENTIELLE DE PÉRICARPE D'AGRUMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2015 ES 201530336**

(43) Date of publication of application:
**24.01.2018 Bulletin 2018/04**

(73) Proprietor: **Suntory Holdings Limited**
**Kita-ku, Osaka-shi,**
**Osaka 530-8203 (JP)**

(72) Inventors:
- **IBUSUKI, Daigo**
  **Kawasaki-shi,**
  **Kanagawa 211-0067 (JP)**
- **FUJIWARA, Masaru**
  **Kawasaki-shi,**
  **Kanagawa 211-0067 (JP)**
- **YOKOO, Yoshiaki**
  **Kawasaki-shi,**
  **Kanagawa 211-0067 (JP)**

(74) Representative: **Vossius & Partner**
**Patentanwälte Rechtsanwälte mbB**
**Siebertstrasse 3**
**81675 München (DE)**

(56) References cited:

| | |
|---|---|
| **JP-A- H07 165 547** | **JP-A- 2000 350 571** |
| **JP-A- 2007 112 892** | **JP-A- 2008 061 511** |
| **US-A- 4 608 266** | **US-A1- 2010 159 115** |

- **POIANA, M. ET AL.: 'Supercritical carbon dioxide (SC-CO2) extraction of grapefruit flavedo.' FLAVOUR AND FRAGRANCE JOURNAL vol. 13, no. 2, 1998, pages 125 - 130, XP055312213**
- **LIU, Y. ET AL.: 'Water-based extraction of pectin from flavedo and albedo of orange peels.' CHEMICAL ENGINEERING JOURNAL vol. 120, no. 3, 2006, pages 203 - 209, XP028035985**
- **YASUHIRO OKUBO: 'Production Technology and Feature of Functional Emulsion, using Food Emulsifier' THE FOOD INDUSTRY vol. 56, 2013, pages 66 - 73, XP009505923**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 272 227 B1

## Description

### TECHNICAL FIELD

The present invention relates to liquid compositions containing essential oils, or essential oils obtained from peel of citrus fruits. More particularly, the present invention relates to liquid compositions that comprise essential oils from peel and specific concentration ratios of limonin and rutin and which are sufficiently low in acidity and bitterness to be capable of inclusion within drinks.

### BACKGROUND ART

Citrus juice obtained by squeezing citrus fruits may sometimes have problems in terms of bitterness, acidity, astringency, unpleasant smells, and so on. Moderate bitterness or acidity may be accepted by consumers as a taste that reminds them of natural fruits, but excessive bitterness or acidity may sometimes make them unpleasant. To deal with the problems, there have been proposed methods such as reducing the excessive bitterness or acidity of citrus juice by addition of a hesperidin glycoside (Patent Document 1) and suppressing bitterness of limonin and the like by addition of citric acid and an alkali metal citrate or addition of condensed phosphate (Patent Documents 2 and 3). Further, there has been proposed a method for controlling the aroma and flavor of citrus juice comprising controlling the concentrations of limonin and polymethoxylated flavones to levels below the threshold taste level thereof and controlling the Brix-acid ratio of the juice therein (Patent Document 4).

Patent Document 5 discloses a specific process for forming a citrus juice extender substantially from natural components of citrus fruit wherein flavedo from the citrus fruit is comminuted and combined with an acidifier such as citric acid and sodium citrate as a buffering agent, sufficient water being added to maintain liquidity of the combination.

Patent Document 6 describes the extraction of flavonoid citrus extract from the byproduct of a juice extraction process, to provide a flavonoid-rich citrus extract.

Patent Document 7 relates to a manufacturing process comprises slicing off only epicarps with oil cells from the residues of citrus fruits after the juices are squeezed, cutting them into fine pieces and distillating them after adding water, to obtain essential oils.

It has been reported that limonin and naringin, which are bitter components of citrus fruits, are highly contained in the peel of a citrus fruit and the part that is growing (the center part of the fruit) (Non-Patent Documents 1 and 2). In this connection, a method has been developed for squeezing juice in such a way that the extracts of these parts are not included in the juice.

### CITATION LIST

#### PATENT DOCUMENTS

- Patent Document 1: JP H11-318379 A
- Patent Document 2: JP S60-246325 A
- Patent Document 3: JP 2013-33 A
- Patent Document 4: JP 2011-500028 A
- Patent Document 5: US 4 608 266 A
- Patent Document 6: US 2010/159115 A1
- Patent Document 7: JP 2007 112 892 A

#### NON-PATENT DOCUMENTS

- Non-Patent Document 1: J. Agric. Food Chem., Vol.45, 2876-2883 1997
- Non-Patent Document 2: Kasetsart J. (Nat. Sci.) 43 : 28 - 36 (2009)

SUMMARY OF INVENTION

TECHNICAL PROBLEM

In the case of producing drinks using the juice of citrus fruits having a strong bitter taste, there is a problem that the amount of juice that can be used in drinks is limited and thus it is difficult to fully reproduce the aroma of the fruit. Another problem is that if bitter components are removed from fruit juice, other components (e.g., aroma components) may also be lost, so not only is the aroma strength low but also the aroma and flavor of the juice may become different from those of natural fruits. Further, the essential oils of the peel of citrus fruits have a tendency to deteriorate in fragrance as soon as they are added to fruit juice, so not only are they unable to maintain fresh aroma but the aroma strength is also low. An object of the present invention is to provide liquid compositions comprising essential oils from peel, having a natural and fresh fragrance derived from natural citrus fruits, being low in irritating bitterness, and being capable of inclusion within drinks.

SOLUTION TO PROBLEM

As a result of extensive research, the inventors found that a liquid composition that was obtained through a process of reducing rutin contained in the peel of citrus fruit and then crashing the peel within water had a fragrance characteristic of citrus essential oils from peel even though it did not contain fruit juice. The inventors have completed this invention by finding out that a liquid composition in which the concentration of rutin with respect to acidity was limited to a certain value did contain a specified amount of limonin but, nonetheless, the liquid composition was low in bitterness and acidity, had an aroma closer to that of natural fruits, and was suitable for inclusion in drinks.
Specifically, the liquid composition:

comprises an essential oil from peel, limonin, and rutin; wherein
the content of the essential oil from peel is in the range of 0.2-3.5% by volume based on the total amount of the composition;
the concentration of the limonin with respect to acidity is in the range of 10-300 mg/kg/Acid;
the concentration of the rutin with respect to acidity is in the range of 10-50 mg/kg/Acid;
the rutin to limonin weight ratio (rutin/limonin) is 1 or less;
the acidity is 2.0% or less;
wherein the essential oil from peel is obtained by using one or more citrus fruits having juice with an acidity of at least 2.0%; and
wherein the liquid composition is obtained by a method comprising removing the super surface layer by slicing the peel thinly enough not to rupture oil sacs in the peel.

The above-described essential oil from peel, limonin, and rutin may be derived from one or more citrus fruits. The above-described liquid composition may be used as aroma juice which is added to drinks and it has a by far lower acidity than conventional types of fruit juice (including comminuted juice). It should be noted here that "mg/kg/Acid" as the unit of the concentrations of limonin and rutin is obtained by dividing the concentration of limonin or rutin (mg/kg) by the acidity of the composition. The acidity as used herein is determined by first measuring the content of organic acids in a liquid by neutralization titration with sodium hydroxide, calculating the same in terms of citric acid, and expressing the converted value in percentage.

The present inventors found that the irritating, intensive bitterness of citrus fruit was high in the super surface layer of the peel (within 1 mm from the outer surface, in particular, within 0.7 mm). By removing the super surface layer by slicing the peel thinly enough not to rupture oil sacs in the peel, the inventors has succeeded in removing the bitterness of the peel while maintaining the aroma components of citrus fruit. Measuring the change in components of the peel before and after removal of the super surface layer, the inventors surprisingly found that the concentration of limonin known as a bitterness component did not change while the concentration of rutin was significantly decreased. From this result, the inventors inferred that rutin has an effect of increasing bitterness and studied the impact of rutin on bitterness. As the result of the studies, the inventors have found that rutin itself has little bitterness but that, in a case where limonin is present together with rutin having a certain proportion or more, the intense bitterness of limonin is increased by rutin. More specifically, the inventors have found that the bitterness of limonin is more intense if the rutin to limonin weight ratio (rutin/limonin) exceeds 1. In the liquid composition of the present invention, the intense bitterness of limonin can be reduced by lowering the rutin/limonin ratio to 1 or less.

The inventors also have found that the bitterness of limonin is increased under high acidity conditions. In the liquid composition of the present invention, the bitterness of limonin can be further reduced by adjusting the acidity to be 2.0% or less.

As will be described below, a liquid composition having a specified amount of essential oil from peel, a specified rutin/limonin ratio, and an acidity of 2.0 or less may be produced, for example, by use of citrus fruit peel from which the super surface layer is removed in a thickness such that oil sacs are not ruptured (i.e., the rutin-reduced citrus fruit peel) and a solvent such as water. When the rutin concentration in fruit peel is not so high, a liquid composition having the specified rutin/limonin ratio may also be obtained by adjusting the mixing ratio of water and the peel, without removing the super surface layer.

BRIEF DESCRIPTION OF DRAWINGS

Fig. 1 is a cross section of a citrus fruit.
Fig. 2 shows an enlarged view of a part of Fig. 1. Super surface layer 1 is to be removed in the invention.
Fig. 3 shows photographs of the appearance of citrus fruits before and after removal of the super surface layer. In each photograph, the one on the left is before the removal and the one on the right is after the removal. The photograph on the top shows lemon, the second is lime, the third is white grapefruit, and the bottom is orange.

DESCRIPTION OF EMBODIMENTS

<Essential oils from peel>

The composition of the present invention contains essential oils from peel in amounts ranging from 0.2 to 3.5% by volume based on the total amount of the composition. The essential oils from peel are a class of fragrant compounds that are mainly collected from the peels of citrus fruits. The fruit's essential oils are based on terpene compounds such as monoterpenes and sesquiterpenes and differ from oils and fats that are based on glycerides. Essential oils can be collected from plants by various methods including steam distillation, expression, solvent extraction, unfleurage, maceration, and supercritical extraction. Although the essential oils from peel to be used in the present invention may be obtained by any of these methods, it is preferable to use the essential oil components that are obtained by expression.

The term "expression" as used herein refers to a method in which a physical force is applied to the peel of a fruit so that an essential oil is obtained from oil cells present in the colored portion of the peel. In a known example of the expression process, the peel is mechanically bruised to rupture oil sacs, from which an essential oil is extracted (Florida Citrus Oils, Kesterson, et al., Technical Bulletin 749, December 1971, ppl5-20.) As will be described later, the peel containing an essential oil may be emulsified under crushing in water and this method is also included in the expression process. The citrus peel includes a dark colored flavedo portion and a white fibrous albedo portion, with the flavedo containing a lot of oil sacs containing a large amount of essential oil (see Fig. 1 quoted from "Kajitsu no Jiten (Dictionary of Fruits)", published by Asakura Shoten, 2008, p. 198.) In the present invention, the peel, or the flavedo in particular, is collected and by emulsifying the oil sac-containing peel as it is crushed in water, a liquid composition can be obtained that contains the essential oil from peel. In the process, the peel or flavedo is preferably collected in such a way that the oil sacs are kept intact as much as possible until they are ruptured within water. By maintaining the oil sacs intact until they are emulsified in water, the essential oil in the oil sacs can be prevented from making direct contact with oxygen to reduce the possibility that the aroma components in the essential oil may deteriorate upon oxidation. The thus obtained essential oil from peel is advantageous in that it is less susceptible to heat and oxygen and that, therefore, the aroma components will experience less deterioration. When the cold press method or other conventional technique for obtaining an essential oil from peel is employed, it is necessary to take an essential oil out of oil sacs before the process completes, so its aroma components may deteriorate under direct action of oxygen.

The fruits to be used for obtaining essential oils from peel may be either of the genus Citrus or the genus Fortunella, both of which are generally consumed as food; for attaining maximum effects of the present invention, the genus Citrus is particularly preferred. Specific examples of the genus Citrus include; flavorful acid citruses (Citrus limon, Citrus aurantifolla, Citrus junos, Citrus spaerocarpa, Citrus aurantium, Citrus sudachi, Citrus depressa, Citrus medica, Citrus medica var. sarcodactylus (Buddha's hand), etc.); oranges (Citrus sinensis) (Valencia orange, Navel orange, Blood orange, etc.); grapefruits (Citrus paradisi) (marsh, ruby, etc.); other citruses (Citrus natsudaidai, Citrus hassaku, Citrus tamurana, Citrus grandis x paradisi (Oroblanco), Citrus reticulata cv. Dekopon, etc.); tangors (Citrus iyo, Citrus tankan, Citrus unshiu x sinensis, Citrus sinensis (Harumi), etc.); tangelos (Citrus x tangelo cv. Seminole, Citrus sinensis (Minneola), etc.); buntans (Citrus maxima, Citrus grandis (Banpeiyu), etc.); Satsuma mandarins (mandarin orange, Citrus kinokuni, Citrus unshiu, Citrus reticulata cv. Ponkan, Citrus tachibana, etc.) The genus Fortunella includes Fortunella crassifolia, Fortunella japonica, Fortunella margarita, etc. In particular, those citrus fruits whose juice itself is so strong in acidity or bitterness that it cannot be incorporated in large quantities in drinks are used in the present invention, namely citrus fruits having juice with an acidity of at least 2.0%, preferably at least 3.0%, and more preferably at least 4.0%.

Citrus fruits having very low sugar contents compared to acidity are also preferred in the present invention since they present a relatively strong perceivable sour taste. Examples of such citrus fruits having very low sugar contents compared to acidity include those having a Brix-acid ratio of 10 or less, preferably 8 or less, more preferably 5 or less, and particularly preferably 3 or less. The acidity as used herein is determined by first measuring the content of organic acids such as citric acid and malic acid by neutralization titration with sodium hydroxide, calculating the same in terms of citric acid, and expressing the converted value in percentage. The Brix-acid ratio refers to degrees Brix (a value indicated in percentage upon measurement with a sugar refractometer) as divided by acidity. Table 1 lists the degrees Brix, acidity and Brix-acid ratio for typical citrus fruits. Fruits that are advantageous for the present invention are exemplified by flavorful acid citruses such as Citrus limon, Citrus aurantifolia, Citrus sudachi, Citrus sphaerocarpa, Citrus depressa, and Citrus junos.

[Table 1]

| <Acidity and degrees Brix of citrus fruits> | | | |
|---|---|---|---|
| Citrus Types | °Brix (%) | Acidity (%) | Brix-acid ratio |
| Citrus limon | 7.00 | 4.50 | 1.56 |
| Citrus aurantifolia | 9.10 | 6.00 | 1.52 |
| Citrus junos | 8.90 | 4.28 | 2.08 |
| Citrus sphaerocarpa | 9.10 | 4.40 | 2.07 |
| Citrus sudachi | 8.20 | 6.63 | 1.24 |
| Citrus depressa | 9.20 | 4.18 | 2.20 |
| Citrus sinensis | 11.0 | 0.80 | 13.75 |
| Citrus paradisi | 9.90 | 1.36 | 7.28 |
| Citrus natsudaidai | 11.1 | 1.51 | 7.35 |
| Citrus tamurana | 10.2 | 1.88 | 5.43 |
| Citrus reticulata cv. Dekopon | 14.4 | 1.39 | 10.36 |
| Citrus iyo | 12.1 | 1.05 | 11.52 |
| Citrus unshiu × sinensis | 12.0 | 0.97 | 12.37 |
| Citrus maxima | 10.3 | 1.13 | 9.11 |
| Citrus unshiu | 10.2 | 0.72 | 14.17 |
| Citrus reticulata cv. Ponkan | 10.0 | 0.73 | 13.70 |

The concentration of the essential oil in the liquid composition ranges from 0.2 to 3.5% by volume based on the total amount of the composition. Preferably, it ranges from 0.3 to 3.0% by volume, more preferably from 0.4 to 2.5% by volume, even more preferably from 0.4 to 2.0% by volume, and particularly preferably from 0.4 to 1.5% by volume. The concentration of the essential oil from peel in the composition can be measured with an essential oil testing apparatus, or a distillation system using an essential oil quantifying device, as will be described below in the Examples.

<Limonin>

The composition of the present invention comprises limonin. Limonin is known to be present in fruits in the form of glycoside (limonin glucoside; Formula 1) or a form obtained by removing sugar from glycoside (limonoate A-ring lactone; Formula 2). Neither of Limonin glucoside and limonoate A-ring lactone presents bitterness. It is known that, after juice has been squeezed from fruits, limonoate A-ring lactone is closed under the acidic condition of the juice and converted into limonin (7,16-dioxo-7,16-dideoxylimondiol, $C_{26}H_{30}O_8$; Formula 3), which produces intense bitterness.

[Formula 1]

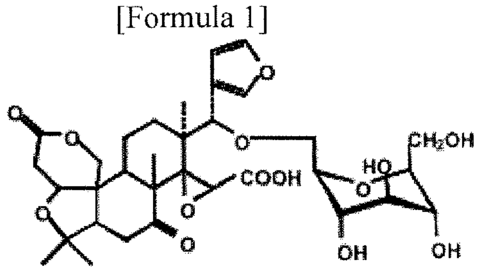

Formula 1

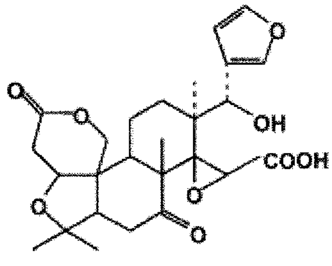

Formula 2

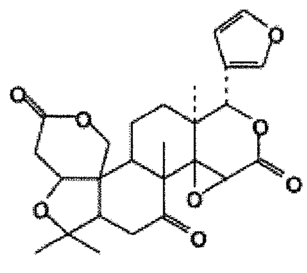

Formula 3

The concentration of limonin in the composition of the present invention is preferably in the range of 10-300 mg/kg/Acid, more preferably in the range of 15-200 mg/kg/Acid, and even more preferably in the range of 20-150 mg/kg/Acid. The unit "mg/kg/Acid" derives from the concentration of limonin in the composition (mg/kg) as a result of dividing it by the acidity of the composition. The acidity as used herein is determined by first measuring the content of organic acids in the composition by neutralization titration with sodium hydroxide, calculating the same in terms of citric acid, and expressing the converted value in percentage. In the present invention, the concentration of limonin in the composition refers to the concentration of the substance of Formula 3.

The concentration of limonin in the liquid composition can be adjusted by adding a commercially available limonin into the composition. Alternatively, as will be understood from the method to be described later, the same result can be obtained by emulsifying the essential oil-containing peel or flavedo as they are crushed within water so that the fruit-derived limonin is extracted into the liquid composition. The limonin concentration in the liquid composition can be measured by use of LC-MS, as will be described later in the Examples.

The limonin in the liquid composition of the present invention is preferably derived from citrus fruits although this is not the sole example of limonin that can be used in the present invention. By using the peel-derived limonin and combining it with the essential oil from peel, there would be obtained a composition whose aroma and flavor is even closer to that of natural citrus fruits.

<Rutin>

The composition of the present invention comprises rutin. The concentration of rutin in the composition of the present invention is in the range 10-50 mg/kg/Acid, preferably in the range of 15-45 mg/kg/Acid. By incorporating rutin in the liquid composition at a certain range of concentration, the limonin bitterness can be reduced. However, in a case where limonin is present together with rutin having a certain proportion or more, the intense bitterness of limonin may be even increased by rutin. The rutin to limonin weight ratio (rutin/limonin) is preferably 1 or less, more preferably 0.8 or less, yet more preferably 0.6 or less. The term "intense bitterness" as used herein refers to a physical stimulus caused by shrinkage of the mucosa of tongue and represents excessive or unpleasant bitterness which differs from moderate bitterness acceptable as a chemical stimulus. In general, it is known that bitter substances in small amounts, not greater than their thresholds, provide foods and dishes with complex, rich flavors and tastes. It is also known that bitterness has a contrast effect with sweetness. However, the bitterness of limonin in the peel and others of citrus fruit is an irritating, excessive bitterness, which is often perceived as unpleasant. This invention may selectively reduce this "intense bitterness" by suppressing the rutin to limonin ratio to be 1 or less.

Rutin (quercetin-3-glucoside, molecular formula $C_{27}H_{30}O_{16}$) is a type of flavone glycosides and is known to be contained in soba (Fagopyrum exculentum), asparagus, etc., and it is also known to be contained in the peel and others of citrus fruits. The rutin in the liquid composition of the present invention is preferably derived from citrus fruits although this is not the sole example of rutin that can be used in the present invention. The concentration of rutin can be adjusted by adding a certain amount of commercially available rutin into the composition containing essential oil from peel and limonin or removing an appropriate amount of rutin from the composition. As will be understood from the method to be described later, the liquid composition may be obtained by emulsifying the essential oil-containing peel or flavedo as they are crushed within water so that the peel-derived essential oil and limonin are extracted into the liquid composition.

In this case, usually, a large amount of rutin is also extracted into the liquid composition, so an appropriate amount of rutin needs to be removed to limit the rutin/limonin ratio to lie within the above-mentioned ranges. In this regard, the present inventors have found a convenient method for limiting the rutin/limonin ratio in the composition to lie within the above-mentioned ranges, namely, they have found that the concentration of rutin can be reduced by removing the super surface layer of peel very thinly (at such a thickness that oil sacs are kept intact) before crushing the peel or flavedo within water. Although it was already known that rutin is contained in the peel of citrus fruits, the present inventors first found out that the content of rutin is high, in particular, near the outer surface of the peel. The rutin concentration in the liquid composition can be measured by use of LC-MS, as will be described later in the Examples.

<Acidity>

If the essential oils from peel of citrus fruits are placed under high acidity conditions, they will readily deteriorate to smell like chemicals. The composition of the present invention, being reduced in acidity, can be used as an aroma composition that is natural and fresh with less deterioration. The composition of the present invention has an acidity of 2.0% or less, preferably 0.1-1.5%, more preferably 0.2-1.2%, and even more preferably 0.2-1.0%. The present inventors found that the intense bitterness of the composition could be reduced further effectively by adjusting the acidity of the composition to lie within the above-stated ranges, in addition to lowering the rutin/limonin ratio to 1 or less. As stated above, the "acidity" as used herein is determined by first measuring the content of organic acids such as citric acid and malic acid by neutralization titration with sodium hydroxide, calculating the same in terms of citric acid, and expressing the converted value in percentage by weight. The comminuted juice from high acidity fruits has a problem that it cannot be included in large quantities in drinks since the drinks become so sour that they are not easy to drink. In this regard, the liquid composition with low acidity of the present invention is advantageous not only in that the perceivable intense bitterness of limonin is lowered, as described above, but also in that large amounts of aroma components of fruit's essential oils can be incorpoated in drinks because the composition can be incorporated in large amounts in the drinks.

The liquid composition of the present invention having a specified amount of fruit's essential oil, a rutin/limonin ratio of 1 or less, and an acidity of 2.0% or less can be produced, for example, by use of citrus fruit peel from which the super surface layer is removed in a thickness such that oil sacs are not ruptured (i.e., the rutin-reduced citrus fruit peel) and a solvent such as water.

<Liquid composition>

The composition of the present invention is in a liquid state at room temperature under atmospheric pressure. Preferably, the composition comprises water as the main solvent. The composition preferably has a pH of less than 5. The low pH condition is advantageous for suppressing the growth of spoilage microorganisms.

As will be described later in a more specific way, one possible method for producing the liquid composition of the present invention comprises the following procedure: the peel or flavedo having oil sacs containing an essential oil is emulsified as it is crushed in water, whereupon the fruit's essential oil is taken out of the oil sacs into the water and, at the same time, the limonin is extracted from the peel into the water. As a result, the liquid composition can be produced without including any components other than the fruit and water and it serves as a liquid flavoring characterized by "no food additives used." By the term "no additives used" is meant that none of the additives on the "List of Products on the Registry of Existing Additives" specified in the Japan Food Sanitation Act have been "externally" added.

The liquid composition of the present invention may be incorporated into foods and drinks for the purpose of flavoring them. In the case of drinks, for example, the composition may be incorporated at concentrations varying from 0.1 to 15% by weight, preferably from 0.2 to 10% by weight, depending on the flavor to be imparted. Being an aqueous composition that is low in either acidity alone or both acidity and degrees Brix and which is relatively high in the content of essential oil from peel, the liquid composition of the present invention has an advantage in that it can be incorporated into drinks in large enough amounts to ensure that the aroma components of the essential oil from peel are amply contained in the drinks. Particularly in the case of producing drinks with the taste of high acidity citrus fruit, the aroma of the fruit can be fully reproduced within the drinks. The liquid composition of the present invention can also be used to impart the flavor of fruit to black tea and other tea beverages. The drinks in which the liquid composition of the present invention may be used are not particularly limited and may include various types such as alcoholic beverages, alcohol-free beverages, carbonated drinks, juice-containing drinks, and tea-based drinks. It is worth particular mention that if the liquid composition does not contain alcohol (ethanol), it can advantageously be used to flavor alcohol-free drinks. In particular, it can advantageously be used to flavor drinks (non-alcohol drinks) of low juice content which is in the range of about 1-30% by weight, preferably 1-20% by weight, and more preferably 1-10% by weight, and this enables the manufacture of drinks that reproduce the fragrance of natural fruits. By using the liquid composition of the present invention, it is also possible to manufacture drinks that contain no synthetic additives such as synthetic flavors and surfactants.

<Production method>

In the course of producing fruit juices from fruits having different rutin concentrations in peel, the present inventors found that, by adjusting amounts of essential oil from peel, limonin, and rutin, as well as acidity, a liquid composition which does not have perceivable irritating bitterness and has a fresh aroma and flavor close to that of natural fruits can be obtained.

The liquid composition of the present invention can be produced by adjusting the amounts of the essential oil from peel, limonin and rutin, as well as acidity. If desired, the composition can be produced using only a fruit or fruits and water in accordance with the method described below, which is given here for illustrative purposes only and is not the sole example that can be employed. The liquid composition that solely consists of fruit-derived components and water presents an aroma more like a natural fruit and, hence, is preferred.

In the case of a fruit that contains a large amount of limonin in the peel, it is necessary to reduce rutin in the peel. To address this problem, first, the super surface layer of the peel of a citrus fruit is removed by slicing the peel thinly enough not to rupture oil sacs in flavedo. The present inventors found that the content of rutin is high, in particular, in the super surface layer of the peel; that rutin increases the intense bitterness of limonin in the peel; and that, by removing the super surface layer of the peel, the concentration of rutin may be selectively reduced thereby to reduce the intense bitterness of the peel. The super surface layer is portion 1 shown in Fig. 2. In general, the super surface layer has a thickness of within 1 mm from the outer surface of the peel, preferably within 0.9 mm, more preferably within 0.8 mm, yet more preferably within 0.7 mm, depending on types and sizes of fruits, and is thin to a degree that most oil sacs remain without being ruptured. By removing the super surface layer in such a way that the oil sacs are kept intact as much as possible, aroma components in the oil sacs can be maintained while reducing the bitterness of peel. Further, although it is known that the aroma components contained in the essential oil in the oil sacs (peel oil) can be easily oxidized and deteriorated, such deterioration of the essential oil can be reduced by keeping the oil sacs intact as much as possible to prevent the essential oil from directly contacting the atmosphere (oxygen). When removing the super surface layer, it is preferable that more than 50% of the number of oil sacs remain without being ruptured, more preferably 70% or more, and yet more preferably 90% or more. The oil sacs can be observed either with an optical microscope or with the naked eye. The super surface layer of peel is preferably removed in an area which accounts for 50% or more of the total outer surface area of the peel, more preferably, 80% or more, yet more preferably 90% or more. It can also be observed with the naked eye whether the super surface layer was removed. If the super surface layer of peel has been removed, the color of the outer surface of the peel slightly changes. For example, in lemons and oranges, redness is reduced and whiteness and yellowness are slightly increased, or the color becomes greenish (see Fig. 3).

In the process of removing the super surface layer of peel, use of tools such as knives or home peelers is not preferable because oil sacs might be ruptured. As a method for solely slicing the super surface layer without rupturing oil sacs of the peel, it is preferable to use rotating drum peeling machines for root vegetables like potato. These peeling machines are disclosed in JP Patent No. 4497427 specification, JP Patent No. 4247923 specification, JP Utility Model Registration No. 3084921 specification, and others. A rotating drum peeling machine has a pair of cylindrical rotating drums rotating outwards from each other at the top. In the present invention, it is preferable to use a peeling machine without having projections such as blades or nibs on its rotating drums. If a peeling machine having such projections on its rotating drums is used for peeling citrus fruits, oil sacs in the peel might be destroyed. As a rotating drum without such projections, for example, a drum having a perforated surface with many holes may preferably be used.

The foregoing operations are necessary for fruits that contain a large amount of rutin in the super surface layer of the peel, but the operations may be omitted for some types of fruits that contain less rutin in the peel.

Second, the peel is collected from the fruit by a commonly employed technique. The collected peel consists of a dark colored flavedo portion having oil sacs and a white fibrous albedo portion. Containing no oil sacs, the albedo portion of the peel has less aroma components than the flavedo portion does; in addition, depending on the fruit from which it is derived, the albedo portion may present a bitter taste. Thus, when collecting the peel, the flavedo portion may be collected after most of the albedo portion has been removed from the peel. In this process, a small amount of the albedo portion may become included in the flavedo portion. In the process of collecting the peel or flavedo, care is preferably taken to ensure that the oil sacs in the flavedo are least destroyed. By not destroying the oil sacs, the essential oil contained in the oil sacs can be protected from oxidative deterioration.

The collected peel or flavedo is then mixed with water. The mixing ratio of water to peel (by weight) is preferably in the range of from about 0.5:1 to about 2.5:1, more preferably from about 0.6:1 to about 1.8:1, even more preferably from about 0.7:1 to about 1.5:1, and particularly preferably from about 0.7:1 to about 1:1. In this case, a pectin-containing fruit part (e.g. albedo or segment membrane) may be mixed in small amounts. After mixing the peel with water, an apparatus such as a mixer or homogenizer that is capable of dispersing the peel in water while shredding it into pieces may be used to form an emulsion in water of the essential oil contained in the oil sacs in the peel and to extract components such as glycerophospholipids and pectin from the peel into water, whereby the emulsion stability of oil drops (essential oil components) is improved. If the peel or flavedo collected with care being taken to keep the oil sacs intact as much

as possible is mixed with water and the essential oil is emulsified while rupturing the oil sacs within water, direct contact of the essential oil with the atmosphere can be avoided to reduce its deterioration. From the resulting mixture of water and the disrupted peel, the solids content is centrifugally or otherwise removed to give a liquid composition. The thus obtained liquid composition solely consists of water and the fruit components and, in the absence of any off-flavors due to components other than the fruit, it presents an aroma and flavor resembling those of natural fruit. As a further advantage, during the production of the liquid composition, the aroma components will undergo less oxidative deterioration and the produced composition presents a fresh aroma and flavor.

EXAMPLES

On the following pages, several examples of the present invention are given but it should be understood that the present invention is by no means limited to these Examples.

(1) Measurement of degrees Brix

Measurement of degrees Brix (%) was performed with a digital refractometer (manufactured by ATAGO CO., LTD.; Model No. RX-5000$\alpha$) at 20°C.

(2) Measurement of acidity

Ten grams of a liquid composition was diluted to a prescribed volume, thereby making a test solution. A given amount of the test solution was titrated with a 0.1 mol/L sodium hydroxide standard solution using phenolphthalein as a pH indicator and the titratable acidity was calculated by the following formula:

$$\text{Acidity (\%)} = K \times (T - B) \times F \times (100/A) \times (1/W) \times 100$$

K: calculated for citric acid = 0.0064
T: the amount of 0.1 mol/L sodium hydroxide solution used for titration (ml)
B: the amount of 0.1 mol/L sodium hydroxide solution used for titration in the same amount of water (ml)
F: the factor of 0.1 mol/L sodium hydroxide solution
A: the volume of sample taken for titration (ml)
W: the weight of sample taken for preparation (g).

(3) Measurement of the essential oil's content

To measure the essential oil's content in the composition, an essential oil quantifying apparatus was used. A round-bottom flask equipped with a condenser capable of trapping the essential oil was charged with 100 mL of the liquid composition, 2 L of distilled water, and boiling stones; atmospheric distillation was performed under heating at about 100 °C for an hour and the amount of the essential oil (mL) collecting in the trap tube was measured to calculate the content of the essential oil.

(4) Measurement of limonin and rutin

Limonin and rutin were analyzed by use of LC-MS.

(Preparation of samples)

Samples for analysis were prepared by the following methods. First, 10 g of the liquid composition was weighed in a centrifugal glass tube (A). Note that when the liquid composition had a degrees Brix of 10% or more, 5 g was weighed; in the case of 20% or more, 2.5 g was weighed; and in the case of 30% or more, 1 g was weighed; in either case, the weighed liquid composition was diluted to 10 mL with distilled water for liquid chromatography. Subsequently, 20 mL of ethanol for liquid chromatography was added and the mixture was vigorously agitated with a vortex mixer for one minute or longer. When high viscosity prevented effective mixing, vigorous manual shaking was optionally performed. The intimate mixture was subjected to a centrifuge (1620 G x 30 min at 20°C) and the supernatant was transferred into another centrifugal glass tube (B). To the precipitate, 20 mL of ethanol for liquid chromatography was added and after breaking the solids loose enough with a suitable device such as a dispensing spoon, the mixture was vigorously agitated

with a vortex mixer for one minute or longer. After centrifugation with a centrifuge (1620 G x 30 min at 20°C), the supernatant was charged into the centrifugal tube (B). The collected supernatants in the centrifugal tube (B) were further centrifuged (1620 G x 30 min at 20°C) and the resulting supernatant was transferred into a 50-mL measuring flask and diluted with ethanol to the marked line. The well mixed supernatant was further diluted 10-fold with ethanol for liquid chromatography and passed through a preliminarily ethanol-washed PTFE filter (product of Toyo Roshi Kaisha, LTD; ADVANTEC DISMIC-25HP 25HP020AN, with a pore size of 0.20 μm and a diameter of 25 mm) to prepare samples for analysis.

(Conditions for LC analysis)

HPLC apparatus: Nexera XR Series (product of Shimadzu Corporation; equipped with system controller, CBM-20A; feed pump, LC-20ADXR; on-line degasser, DGU-20A3; autosampler, SIL-20ACXR; column oven, CTO-20A; and UV/VIS detector, SPD-20A) Column: CAPCELL CORE AQ (particle size, 2.7 μm; inside diameter, 2.1 mm x 150 mm; product of Shiseido Company, Limited)
Mobile phase A: 0.1% aqueous solution of formic acid
Mobile phase B: acetonitrile
Flow rate: 0.6 mL/min
Density gradient conditions: 0.0-0.5 min (15% B)→6.0 min (25% B), with 10.0 min (75% B) →10.1-11.0 min (100% B), with 3.0 min equilibration by the initial mobile phase Column temperature: 40 °C
Sample injection: injected in a volume of 2.0 μL
Sample charge into mass spectrometer: 1.8-11.0 min
(Conditions for mass spectroscopy)
Mass spectrometer: 4000 Q TRAP (product of AB Sciex)
Ionization method: ESI (Turbo Spray), positive mode
Conditions of ionization chamber: CUR, 10; IS, 5500; TEM, 650; GS1, 80; GS2, 60; ihe, ON; CAD, Medium
Detection method: MRM mode
Detection conditions: (Q1→Q3, DP, CE, CXP, EP):

Rutin (611.2→303.1, 76, 25, 12, 10)
Limonin (471.2→425.1, 101, 29, 12, 10)

Peak detection time: Being subject to confirmation with standard samples, the following data may be given as a guide. Rutin (3.01 min), Limonin (8.80 min)

(Quantification method)

Standard samples were purchased from Wako Pure Chemical Industries, Ltd. At least three standard sample solutions of different concentrations were used and quantification was performed by the absolute calibration method based on the peak areas obtained. In the case of measurements that turned out to give values that were outside the ranges of calibration curves, the factor of dilution with ethanol at the final stage of analysis sample preparation was appropriately adjusted to perform another measurement.

<Example 1>

(1) Production of lemon aroma composition

By use of a rotating drum peeler for root vegetables, the super surface layer of a whole lemon was removed in such a way that 90% or more of oil sacs remained intact. The obtained lemon processed product was cut in halves and most of the pulp, seeds, segment membranes, and albedo was removed from the peel to obtain flavedo from which the super surface layer was removed. The obtained flavedo was mixed with water at a weight ratio of 1:1 and the mixture was milled with a commercial juice mixer, with care being taken to ensure that the mixture would not have a paste-like consistency; after stirring at room temperature for 30 minutes, the mixture was passed through a 40-mesh strainer to effect solid-liquid separation. The liquid phase was thereafter homogenized at 0.2 MPa and the insoluble solids were removed from the resulting suspension by centrifugation (6000 G x 5 min) and then pasteurized by heating at 90°C for one minute to thereby prepare a liquid composition (Invention Product 1). A liquid composition (Comparative Example 1) was produced by the same method as used to produce Invention Product 1, except that the super surface layer of a whole lemon was not removed. Invention Product 1 and Comparative Example 1 were compared with each other and the results are shown in Table 3 below. The various components were measured by the methods described above. In

Invention Product 1 without the super surface layer, the rutin content with respect to acid was greatly reduced.

(2) Sensory evaluation

Invention Product 1 and Comparative Example 1 were tasted by licking them directly and evaluated for their flavor. The flavor attributes evaluated were the intensity of irritating bitterness and the intensity of a natural fresh aroma, and the grading system was on a 5-point scale using the criteria shown in Table 2 below. The results are shown in Table 3. Although the Invention Product had a high concentration of limonin, the irritating bitterness in it was dramatically reduced compared to that in the Comparative Example. This suggested that, by controlling the concentration of rutin with respect to acidity, the concentration of limonin with respect to acidity, and the ratio thereof, a flavoring liquid could be obtained that had a fresh and strong fragrance and yet was free from irritating bitterness.

In addition, lemon taste drinks were produced by use of Invention Product 1 and Comparative Example 1 prepared in (1) above and were evaluated for their flavor. The samples were formulated according to the recipes shown in Table 4 so that the degrees Brix (°Brix) or the contribution of essential oil content could also be compared, and they were filled into bottles and pasteurized by heating at 85°C for 5 minutes to produce lemon taste drinks. The results are shown in Table 4. The lemon taste drink produced by use of the Invention Product did not become so sour that it was not hard to drink, even though the Invention Product was incorporated in a large amount in the drink. Further, this drink had a natural fresh fragrance derived from a natural citrus fruit and yet was free from any perceivable irritating bitterness.

[Table 2]

| Score | Degree |
|---|---|
| 4 | Perceived intensely |
| 3 | Perceived considerably |
| 2 | Perceived moderately |
| 1 | Perceived slightly |
| 0 | Not perceived |

[Table 3]

| | Invention product 1 | Comparative Example 1 |
|---|---|---|
| Degrees Brix (°Brix:%) | 4.2 | 5.7 |
| Acidity (%) | 0.38 | 0.67 |
| Essential oil content (%) | 0.45 | 1.10 |
| Limonin content per acidity(mg/kg/Acid) | 111.8 | 67.1 |
| Rutin content per acidity (mg/kg/Acid) | 47.1 | 92.7 |
| Rutin/limonin ratio | 0.4 | 1.4 |
| Flavor evaluation (Intensity of irritating bitterness) | 1 | 4 |
| Flavor evaluation (Intensity of fresh fragrance) | 3 | 4 |

[Table 4]

| (Unit: g/L) | | | |
|---|---|---|---|
| | 1 | 2 | 3 |
| Aroma composition | Comparative Example 1 | | Invention Product 1 |
| | 7.4 | 4.1 | 10.0 |
| Granulated sugar | 90.0 | 90.0 | 90.0 |

(continued)

| (Unit: g/L) | | | |
|---|---|---|---|
| | 1 | 2 | 3 |
| Citric acid anhydride | 1.5 | 1.5 | 1.5 |
| Purified water | q. s. | q. s. | q. s. |
| Flavor evaluation (Intensity of irritating bitterness) | 4 | 3 | 0 |
| Flavor evaluation (Intensity of fresh fragrance) | 4 | 3 | 3 |

<Example 2>

(1) Production of lemon aroma composition

By use of a variety of kinds of lemons, lemon aroma compositions were prepared in the same method as used to produce Invention Product 1 of Example 1, and analyses were conducted for various components. The results are shown in Table 5.

(2) Sensory evaluation

To solutions adjusted with sucrose and citric acid anhydride to °Brix of 9.0 and an acidity of 0.15%, Invention Products 2 to 11 prepared in (1) above were respectively added in 10 g/L; the samples were then filled into bottles and pasteurized by heating at 85°C for 5 minutes to produce bottled drinks with lemon taste. The obtained drinks were evaluated for their flavor in the same manner as in Example 1. Invention Products 2 to 11 having acidities, rutin concentrations per acidity, limonin concentrations per acidity, and rutin/limonin concentration ratios that were adjusted to lie within specified ranges were suppressed in irritating bitterness and yet had a fresh and pleasant aroma characteristic of citrus fruit.

[Table 5]

| | Invention product 2 | Invention product 3 | Invention product 4 | Invention product 5 | Invention product 6 |
|---|---|---|---|---|---|
| Degrees Brix (°Brix:%) | 4.8 | 4.6 | 4.1 | 4.5 | 4.5 |
| Acidity (%) | 0.63 | 0.63 | 0.57 | 0.67 | 0.57 |
| Essential oil content (%) | 0.70 | 0.65 | 0.45 | 0.55 | 0.80 |
| Limonin content per acidity (mg/kg/Acid) | 96.2 | 68.7 | 43.1 | 42.8 | 52.0 |
| Rutin content per acidity (mg/kg/Acid) | 20.7 | 19.8 | 18.6 | 11.4 | 18.4 |
| Rutin/limonin ratio | 0.2 | 0.3 | 0.4 | 0.3 | 0.4 |

| | Invention product 7 | Invention product 8 | Invention product 9 | Invention product 10 | Invention product 11 |
|---|---|---|---|---|---|
| Degrees Brix (°Brix:%) | 4.1 | 3.9 | 4.1 | 3.9 | 4.2 |
| Acidity (%) | 0.52 | 0.36 | 0.60 | 0.58 | 0.60 |
| Essential oil content (%) | 0.45 | 0.45 | 0.40 | 0.38 | 0.50 |
| Limonin content per acidity (mg/kg/Acid) | 92.8 | 138.5 | 111.8 | 29.7 | 81.7 |
| Rutin content per acidity (mg/kg/Acid) | 17.3 | 39.0 | 47.1 | 17.2 | 13.9 |
| Rutin/limonin ratio | 0.2 | 0.3 | 0.4 | 0.6 | 0.2 |

<Example 3>

By use of lime, a lime aroma composition was prepared in the same method as used to produce Invention Product 1 of Example 1, and analyses were conducted for various components. The results are shown in Table 6. In the same manner as in Examples 1 and 2, the sensory evaluation was performed for the composition and a bottled drink containing the composition, and the evaluation showed that the composition and the drink had suppressed irritating bitterness and had a fresh and pleasant aroma characteristic of citrus fruit, and thus were suitable for a flavoring liquid and a bottled drink, respectively.

[Table 6]

| | Invention Product 12 |
|---|---|
| Acidity (%) | 0.79 |
| Essential oil content (%) | 0.35 |
| Limonin concentration per acidity (mg/kg/Acid) | 285.1 |
| Rutin concentration per acidity (mg/kg/Acid) | 15 |
| Rutin/limonin ratio | 0.05 |

<Example 4> (Reference)

A flavedo fraction obtained from a whole lemon without removing the super surface layer of the lemon was mixed with water and emulsified to prepare a composition, and the components of the obtained composition were analyzed. Table 7 shows the results. In the same manner as in Examples 1 and 2, the sensory evaluation was performed for the composition and a bottled drink containing the composition, and the evaluation showed that the composition and the drink had irritating bitterness, but were suitable for a flavoring liquid and a bottled drink, respectively.

[Table 7]

| | Product 13 |
|---|---|
| Acidity (%) | 0.54 |
| Essential oil content (%) | 0.85 |
| Limonin concentration per acidity (mg/kg/Acid) | 128.6 |
| Rutin concentration per acidity (mg/kg/Acid) | 15.1 |
| Rutin/limonin ratio | 0.12 |

## Claims

1. A liquid composition comprising an essential oil from peel, limonin, and rutin,
   wherein a content of the essential oil from peel is in a range of 0.2-3.5% by volume based on the total amount of the composition;
   wherein a concentration of the limonin with respect to acidity is in the range of 10-300 mg/kg/Acid as defined in the description
   wherein a concentration of the rutin with respect to acidity is in the range of 10-50 mg/kg/Acid;
   wherein a rutin to limonin weight ratio (rutin/limonin) is 1 or less;
   wherein the acidity is 2.0% or less;
   wherein the essential oil from peel is obtained by using one or more citrus fruits having juice with an acidity of at least 2.0%; and
   wherein the liquid composition is obtained by a method comprising removing the super surface layer (within 1 mm from the outer surface) by slicing the peel thinly enough not to rupture oil sacs in the peel.

2. The liquid composition according to claim 1, wherein the limonin is derived from one or more fruits.

3. The liquid composition according to claim 1 or 2, wherein the rutin is derived from one or more fruits.

4. The liquid composition according to any one of claims 1 to 3 obtained by use of a solvent and a citrus fruit peel from which a super surface layer has been removed in a thickness such that oil sacs are not ruptured, wherein the citrus fruit is selected from one or more citrus fruits having juice with an acidity of at least 2.0%.

5. A drink comprising the liquid composition according to any one of claims 1 to 4 at a concentration in a range of 0.1-15% by weight based on the total weight of the drink.

## Patentansprüche

1. Eine flüssige Zusammensetzung, umfassend ein ätherisches Öl aus Schale, Limonin und Rutin,
   wobei ein Gehalt des ätherischen Öls aus der Schale in einem Bereich von 0,2 - 3,5 Volumen-%, bezogen auf die Gesamtmenge der Zusammensetzung, liegt;
   wobei eine Konzentration des Limonins hinsichtlich des Säuregehalts im Bereich von 10 - 300 mg/kg/Säure, wie in der Beschreibung definiert, liegt;
   wobei eine Konzentration des Rutins hinsichtlich des Säuregehalts im Bereich von 10 - 50 mg/kg/Säure liegt;
   wobei das Gewichtsverhältnis von Rutin zu Limonin (Rutin/Limonin) 1 oder weniger beträgt;
   wobei der Säuregehalt 2,0% oder weniger beträgt;
   wobei das ätherische Öl aus der Schale unter Verwendung einer oder mehrerer Zitrusfrüchte, deren Saft einen

Säuregehalt von mindestens 2% aufweist, erhalten wird; und
wobei die flüssige Zusammensetzung unter Verwendung eines Verfahrens, umfassend das Entfernen der obenliegenden Oberflächenschicht (innerhalb 1 mm ab der äußeren Oberfläche) durch Schneiden der Schale derart dünn, dass die Ölsäcke in der Schale nicht reißen, erhalten wird.

**2.** Die flüssige Zusammensetzung gemäß Anspruch 1, wobei das Limonin aus einer Frucht oder mehreren Früchten stammt.

**3.** Die flüssige Zusammensetzung gemäß Anspruch 1 oder 2, wobei das Rutin aus einer Frucht oder mehreren Früchten stammt.

**4.** Die flüssige Zusammensetzung gemäß einem der Ansprüche 1 bis 3, erhalten unter Verwendung eines Lösungsmittels und einer Zitrusfruchtschale, von welcher eine obenliegende Oberfläche in einer Dicke entfernt wurde, so dass die Ölsäcke nicht reißen, wobei die Zitrusfrucht ausgewählt ist aus einer oder mehreren Zitrusfrüchten, deren Saft einen Säuregehalt von mindestens 2,0 % aufweist.

**5.** Ein Getränk, umfassend die flüssige Zusammensetzung gemäß einem der Ansprüche 1 bis 4 in einer Konzentration in einem Bereich von 0,1 - 15 Gew.-%, bezogen auf das Gesamtgewicht des Getränks.

## Revendications

**1.** Composition liquide comprenant une huile essentielle d'écorce, de la limonine et de la rutine,
où une teneur de l'huile essentielle d'écorce est dans une plage de 0,2-3,5% en volume sur la base de la quantité totale de la composition;
où une concentration de la limonine par rapport à l'acidité est dans la plage de 10-300 mg/kg/acide telle que définie dans la description,
où une concentration de la rutine par rapport à l'acidité est dans la plage de 10-50 mg/kg/acide;
où un rapport en poids de la rutine à la limonine (rutine/limonine) est 1 ou moins;
où l'acidité est 2,0% ou moins;
où l'huile essentielle d'écorce est obtenue en utilisant un ou plusieurs agrumes ayant un jus avec une acidité d'au moins 2,0%; et
où la composition liquide est obtenue par un procédé comprenant le retrait de la couche superficielle supérieure (à moins de 1 mm de la surface extérieure) en coupant l'écorce en lamelles suffisamment finement pour ne pas rompre les vésicules lipidiques dans l'écorce.

**2.** Composition liquide selon la revendication 1, où la limonine est issue d'un ou plusieurs fruits.

**3.** Composition liquide selon la revendication 1 ou 2, où la rutine est issue d'un ou plusieurs fruits.

**4.** Composition liquide selon l'une quelconque des revendications 1 à 3 obtenue en utilisant un solvant et une écorce d'agrume dont une couche superficielle supérieure a été retirée sur une épaisseur telle que les vésicules lipidiques ne soient pas rompues, où l'agrume est choisi parmi un ou plusieurs agrumes ayant un jus avec une acidité d'au moins 2,0%.

**5.** Boisson comprenant la composition liquide selon l'une quelconque des revendications 1 à 4 à une concentration dans une plage de 0,1-15% en poids sur la base du poids total de la boisson.

Fig. 1

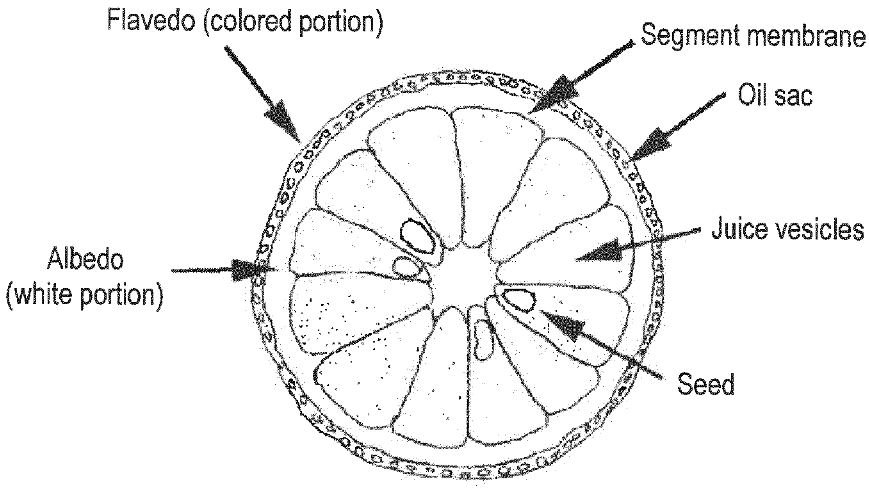
Flavedo (colored portion)
Segment membrane
Oil sac
Juice vesicles
Albedo
(white portion)
Seed

Fig. 2

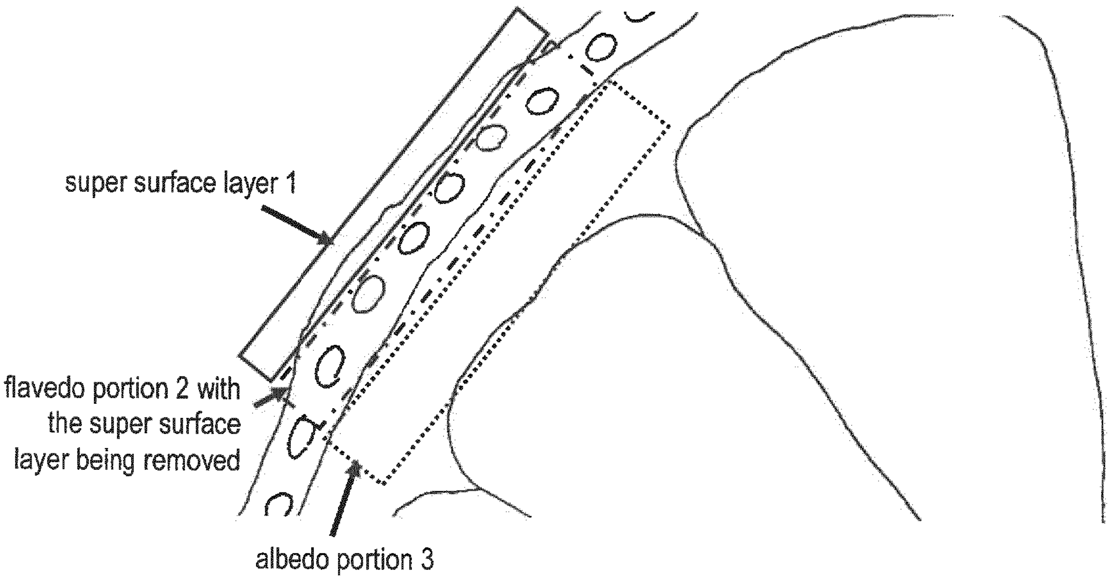
super surface layer 1
flavedo portion 2 with
the super surface
layer being removed
albedo portion 3

Fig. 3

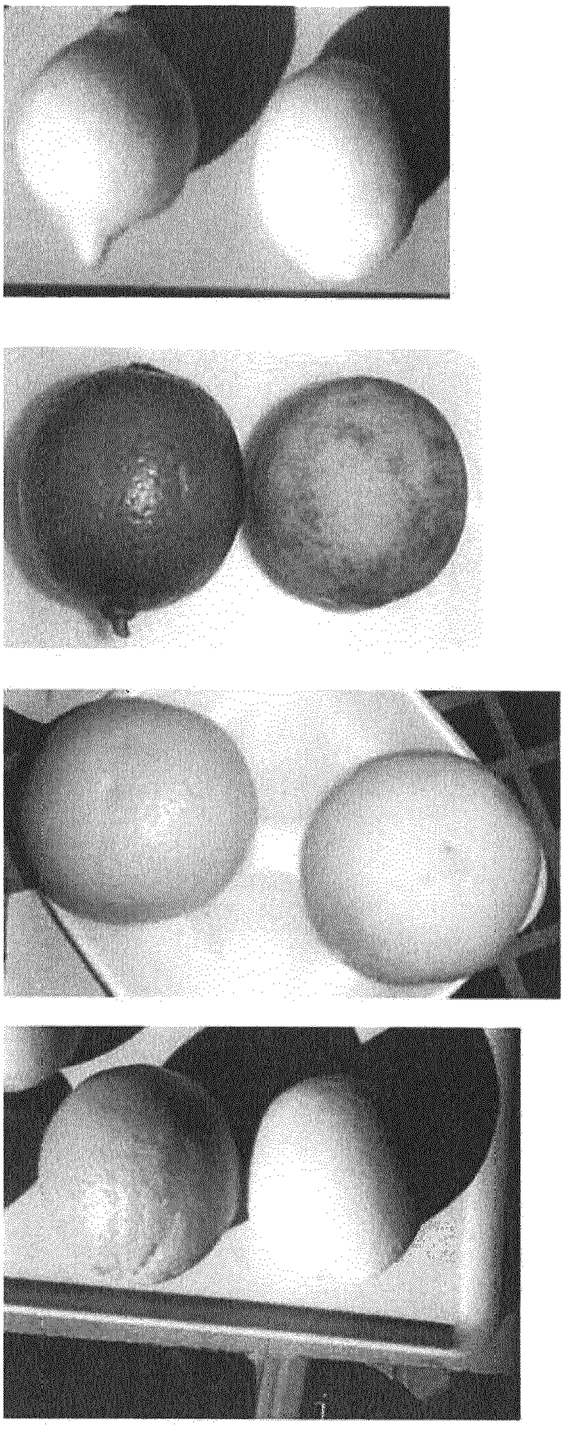

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11318379 A
- JP 60246325 A
- JP 2013000033 A
- JP 2011500028 A
- US 4608266 A
- US 2010159115 A1
- JP 2007112892 A
- JP 4497427 B
- JP 4247923 B
- JP 3084921 U


### Non-patent literature cited in the description


- *J. Agric. Food Chem.,* 1997, vol. 45, 2876-2883
- **KASETSART J.** *Nat. Sci.,* 2009, vol. 43, 28-36
- **KESTERSON et al.** Florida Citrus Oils. *Technical Bulletin 749,* December 1971, I5-20
- Kajitsu no Jiten (Dictionary of Fruits). Asakura Shoten, 2008, 198